# EUROPÄISCHE PATENTSCHRIFT

(11) **EP 2 702 943 B1**
(45) Veröffentlichungstag und Bekanntmachung des Hinweises auf die Patenterteilung: **13.01.2016**
(21) Anmeldenummer: 13003742.7
(22) Anmeldetag: 26.07.2013
(51) Int. Cl.: A61B 5/15, A61B 5/153, B65D 41/04, F16L 37/244

(54) **VORRICHTUNG ZUR ENTNAHME VON KÖRPERFLÜSSIGKEITEN UND VERFAHREN ZUM MONTIEREN EINER SOLCHEN VORRICHTUNG**
DEVICE FOR WITHDRAWING BODY LIQUIDS AND METHOD FOR FITTING SUCH A DEVICE
DISPOSITIF DE PRÉLÈVEMENT DE LIQUIDES CORPORELS ET PROCÉDÉ DE MONTAGE D'UN TEL DISPOSITIF

(30) Priorität: 27.08.2012 DE 102012016936
(43) Veröffentlichungstag der Anmeldung: 05.03.2014
(73) Patentinhaber: SARSTEDT AG & Co., 51588 Nümbrecht (DE)
(72) Erfinder: Sarstedt, Walter, 51588 Nümbrecht (DE)
(74) Vertreter: Grosse, Wolf-Dietrich Rüdiger

(56) Entgegenhaltungen:
- EP-A1- 1 161 923
- CA-C- 1 333 463
- DE-U1- 8 530 544
- DE-U1- 29 800 233
- US-A- 3 927 783
- US-A- 6 015 054
- US-A1- 2007 278 173
- US-B1- 6 354 450

## Beschreibung

Die Erfindung betrifft eine Vorrichtung zur Entnahme von Körperflüssigkeiten, insbesondere Blut, mit einer verformungssteifen Führungshülse und einem Entnahmeröhrchen, das an seinem vorderen Ende mit einer Kappe verschlossen ist, die mit einem domartigen Ansatz und einem in diesem angeordnetem durchstechbaren Stopfen für die aufsteckbare Führungshülse versehen ist, die auf der dem Ansatz zugewandten Seite eine von einem sich beim Aufstecken der Führungshülse oberhalb des Ansatzes harmonikaartig zusammendrückenden Ventilgummi umschlossene Kanüle und auf der dem Ansatz abgewandten Seite ein Verbindungsstück oder das vordere Ende der Kanüle, insbesondere Doppelkanüle, aufweist, wobei der Ansatz und die Führungshülse mit sich zu einer der Rückstellkraft des beim Aufstecken harmonikaartig zusammengedrückten Ventilgummis widerstehenden Verriegelung der Führungshülse auf dem Ansatz ergänzenden Mitteln ausgebildet sind. Weiterhin betrifft die Erfindung ein Verfahren zum Montieren einer derartigen Vorrichtung.

Solche Vorrichtungen werden sowohl zur Blutentnahme aus einem Blutgefäß oder aus einem Blutspendebeutel als auch zur Probennahme aus einem Sammelbehälter für beispielsweise Urin benötigt. Hierbei tritt in allen Fällen die Schwierigkeit auf, dass das die Kanüle umschließende bzw. einhüllende, sich beim Zusammenfügen bzw. Aufstecken der Führungshülse auf den in der Regel zylindrischen domartigen Ansatz ziehharmonikaartig zusammenschiebende Ventilgummi eine der Haltekraft zwischen Führungshülse und Dom entgegenwirkende Feder- bzw. Rückstellkraft ausübt, die dazu führen kann, dass sich die Führungshülse von dem Dom bzw. zylindrischen Ansatz der Kappe abdrückt. Um dies zu vermeiden, befassen sich zahlreiche Entwicklungen mit Gegenmaßnahmen.

Bei einer aus der DE 30 49 503 C bekannten Blutentnahmevorrichtung der eingangs genannten Art besitzt die das Entnahmeröhrchen an seinem vorderen Ende abschließende Kappe einen zylindrischen, in axialer Richtung vorstehenden Ansatz bzw. Dom. Der Ansatz ist an seinem vorderen Ende durch einen durchstechbaren Verschlussstopfen abgeschlossen, der auf einer mit einer Mittelbohrung versehenen Stirnplatte des Ansatzes aufliegt und von einem am vorderen Ende umgebördelten Kragen gehalten wird. Die zylindrische Führungshülse, die an ihrem vorderen Ende in einem Halter eine doppelendige, beiderseits mit einer Schneidkante versehene Kanüle trägt, deren aus der Führungshülse vorstehendes Ende zur Einführung in eine Vene dient, während ihr hinteres Ende so weit in die Führungshülse hineinragt, dass es beim Ansetzen der Führungshülse an das Entnahmeröhrchen den Verschlussstopfen durchsticht, ist axial verschiebbar und drehbar auf dem Ansatz angeordnet. Das hintere, in die Führungshülse hineinragende Kanülenende wird von einem sackartigen Ventilgummi solcher Länge eingehüllt, dass die Schneidkante des hinteren Kanülenendes bei gestrecktem Ventilgummi noch nicht dessen Boden berührt.

Damit die Führungshülse trotz der Federkraft des Ventilgummis in ihrer Position auf dem Ansatz gehalten wird, ist der Ansatz zur Verbindung der Doppelkanüle mit einem seitlich vorstehenden Haltenocken versehen, dem winkelartige Schlitzausnehmungen in der Führungshülse zugeordnet sind. Mittels des in einen der Schlitze eingeführten Haltenockens lässt sich eine bajonettverschlussartige Drehverriegelung erreichen, bei der die Führungshülse in lockerer Passung auf dem Ansatz sitzt. Eine solche Drehverriegelung gewährleistet einen sehr sicheren Zusammenhalt der zusammengefügten Teile der Blutentnahmevorrichtung, erhöht allerdings deren Herstellungsaufwand. Außerdem setzt das Ankuppeln bzw. Aufstecken und Verriegeln der Führungshülse voraus, dass der Haltenocken zunächst durch Drehen des Entnahmeröhrchens mit der verschließenden Schraub- oder Stopfenkappe in Flucht mit einem der Einführschlitze gebracht werden muss, was eine suchende Handhabung erfordert, weil die Teile gezielt gegeneinander ausgerichtet werden müssen.

Eine stabile Verbindung zwischen der Führungshülse und dem Ansatz dieser bekannten Blutentnahmevorrichtung erfordert, wie sich gezeigt hat, mehrere von dem Ansatz seitlich abstehende Vorsprünge bzw. Haltenocken. Beim Herstellen von Formteilen durch Spritzgießen bereitet in der Regel die Entformung des fertig gespritzten Teiles bzw. Produktes nicht unerhebliche Probleme. Dies vor allem dann, wenn das Spritzgussteil mehrere Vorsprünge aufweist, von denen mindestens zwei diametral zueinander angeordnet sind bzw. dessen Herstellung ein Formnest bzw. einen -hohlraum in mehr als einer Formbacke erfordert. Die große Problematik einer Mehrfach-Nockenanordnung beschreibt die EP 0 818 296 B1 und schlägt deshalb vor, entweder alle oder zumindest einige der Vorsprünge nicht mehr vollständig, sondern nur zur Hälfte auszubilden und damit beispielsweise zwei zueinander im wesentlichen diametral angeordnete Teilkavitäten zu ermöglichen, die im Vergleich zu einer vollständigen Kavität jeweils nur zur Hälfte ausgebildet sind und sich ausschließlich in den beweglichen Formbacken vorsehen lassen.

Durch die EP 1 455 650 B1 sind mehrere Lösungen bekannt geworden, die den Herstellungsaufwand verringern und bei gleichzeitig einfacherer Handhabung eine Haltekraft zwischen Führungshülse und Ansatz ermöglichen, die größer ist als die entgegenwirkende Rückstellkraft des Ventilgummis. Diese wie auch aus der DE 44 02 690 C2 bekannte, weitere Verriegelungen beruhen darauf, dass zumindest ein Teil der Verriegelungs- bzw. Haltemittel nachgiebig oder ausweichend ausgebildet ist, um die Führungshülse kraftschlüssig auf dem Entnahmeröhrchen festzulegen.

Der Erfindung liegt die Aufgabe zugrunde, eine Vorrichtung der eingangs genannten Art zu schaffen, die eine größere Bedienungssicherheit bietet und eine einfachere Herstellung ermöglicht. Weiterhin soll ein Verfahren geschaffen werden, mit dem sich eine solche Vorrichtung zur Vorbereitung der Entnahme der Körperflüssigkeit, insbesondere Blut, in einen betriebsfertigen Zustand montieren bzw. zusammenfügen lässt.

Diese Aufgabe wird mit einer Vorrichtung erfindungsgemäß dadurch gelöst, dass am Außenmantel des domartigen Ansatzes umfangsverteilt radial vorspringende, voneinander beabstandete Außengewindesegmente und am Innenmantel der Führungshülse umfangsverteilt radial vorspringende, einander überlappende Innengewindesegmente ausgebildet sind, wobei über eine Teillänge und parallel zu den Innengewindesegmenten verlaufend taschenartige, stirnendseitig von jeweils einem ersten und einem zweiten Anschlag begrenzte Vertiefungen, in die in der Endlagenposition der Führungshülse die Außengewindesegmente des Ansatzes eingreifen, ausgebildet sind.

Es lässt sich damit eine einfache Konstruktion erreichen, die zur spritzgusstechnischen Herstellung von Führungshülse und Kappe mit Ansatz, wobei die Kappe eine Schraubkappe oder eine Stopfenkappe sein kann, und den nur geringfügig, etwa 1 mm, vorspringenden Segmenten keine Formbacken bzw. Backenwerkzeuge mehr erforderlich macht, so dass das Entformen nicht beeinträchtigt wird und die fertigen Spritzgussteile ungehindert ausgeworfen werden können. Die Herstellung gegenüber einem herkömmlichen Verschluss, z.B. konventionellen Bajonettverschluss, ist damit wesentlich kostengünstiger. Die Führungshülse lässt sich zudem völlig umfangsgeschlossen ausführen, so dass anders als bei zumindest ausschnittsweise offenen Führungshülsen eventuell vom Ventilgummi abgelöste Bluttröpfchen nicht nach außen gelangen können. Durch die gleichmäßige Verteilung der Funktionspärchen, bestehend einerseits aus den Außengewindesegmenten des domartigen Ansatzes und andererseits den Innengewindesegmenten mit den taschenartigen Vertiefungen der Führungshülse, wird ein sicherer koaxialer Sitz der Führungshülse mit der von ihr getragenen Kanüle auf dem Ansatz der Kappe gewährleistet, wodurch die Anwendung und hierbei insbesondere die Punktion erheblich erleichtert wird.

Weiterhin lässt sich der gewünscht gute Halt der aufgesteckten Führungshülse bei gleichwohl leichtgängigem Aufstecken und Abziehen erreichen, beides verbunden mit einer teilweisen Schraub- bzw. Aufschraubbewegung, um in schonender Weise bei in der Vene verbleibender Kanüle mehrere Entnahmeröhrchen ruckfrei anzukoppeln und Blut in mehrere Entnahmeröhrchen abzufüllen. Denn anders als das beispielsweise bei einer hemmenden Gewindeverbindung, d.h. Außengewinde am domartigen Ansatz und dazu korrespondierendes Innengewinde der Führungshülse, wie für z.B. ein 4-gängiges Gewinde der DE 85 30 544 U1 bekannt, der Fall wäre, wird die Führungshülse zunächst nur auf dem Ansatz geführt und erst dann, wenn der Gegendruck durch das Ventilgummi überwunden und der Stopfen des Ansatzes von der Kanüle durchstochen ist, gleiten die Außengewinde- und Innengewindesegmente mit dann einsetzender Drehbewegung der Führungshülse bis zum Erreichen der in Drehrichtung liegenden, ersten Anschläge der Vertiefungen übereinander. Die geführte lineare bzw. axiale Bewegung geht dabei in eine sanfte Gleit- und sich dann anschließende Drehbewegung ohne entgegenwirkende Durchdringungskraft über. Das gilt so gleichermaßen mit umgekehrtem Bewegungsablauf beim Abziehen bzw. Entfernen der Führungshülse. Die einander überlappende Ausbildung der Innengewindesegmente ermöglicht den Außengewindesegmenten beim Einsetzen der Drehbewegung eine größere, nämlich übergangslose Tragfläche. Die Innengewinde- und die in einem nach dem Durchstechen des Ventilgummis unter dem zusammengedrückten Ventilgummi liegendem Bereich umfangsverteilt vorgesehenen Außengewindesegmente können mit einer gleichen Steigung vorgesehen werden, worunter verstanden wird, dass jedes Außengewindesegment und jedes Innengewindesegment einen in derselben Ebene verlaufenden untersten Ausgangspunkt und obersten Endpunkt besitzt.

Die der Erfindung zugrundeliegende Aufgabe wird mit einem Verfahren erfindungsgemäß dadurch gelöst, dass der domartige Ansatz der Kappe mit einer linearen Bewegung in die Führungshülse eingeschoben wird, bis nach dem Durchstechen des Stopfens des Ansatzes mittels des hinteren, dann aus dem zusammengedrückten Ventilgummi vorstehenden Kanülenendes umfangsverteilt radial vorspringende, voneinander beabstandete Außengewindesegmente am Außenmantel des Ansatzes auf radial vorspringende, einander überlappende Innengewindesegmente am Innenmantel der Führungshülse auftreffen, dass danach mit einer Schraubbewegung der domartige Ansatz und die Führungshülse weiter zusammengefügt werden, wobei die Schraubbewegung von in Drehrichtung liegenden ersten Anschlägen von über eine Teillänge und parallel zu den Innengewindesegmenten verlaufenden taschenartigen Vertiefungen begrenzt wird und mit dem Ende der Drehbewegung unter der Kraft des zusammengedrückten, komprimierten Ventilgummis die Führungshülse entgegen der Richtung der Aufschraubbewegung linear auf dem domartigen Ansatz zurückgeschoben wird, wobei die Außengewindesegmente in die Vertiefungen eingerastet und in dieser Position durch den ersten Anschlägen am anderen Stirnende der Vertiefungen gegenüberliegenden zweiten Anschlägen gegen ein Zurückdrehen oder Herausrutschen gesichert werden.

Die Außengewinde- und Innengewindesegmente sind so ausgelegt, dass ein Überdrehen mit normal aufzuwendender Kraft nicht möglich ist, wobei der Anwender deutlich verspürt, dass der in Drehrichtung der Aufschraubbewegung liegende erste Anschlag erreicht wurde. Wenn mit dem Ende der Drehbewegung die Führungshülse durch die Kraft des sich etwas entspannenden, zuvor komprimierten Ventilgummis entgegen der Aufschraubrichtung zurückgeschoben wird, kommen die Außengewindesegmente in den taschenartigen Vertiefungen in einer gesicherten Stellung zur Ruhe. Wenn der Anwender diese Verriegelungsposition lösen will, braucht er die Führungshülse entsprechend dem geringen Rückstellweg durch das Ventilgummi nur wieder etwas weiter auf den domartigen Ansatz aufzuschieben, so dass die Außengewindesegmente aus den taschenartigen Vertiefungen herausrutschen. Anschließend lässtsich durch eine der Drehrichtung beim Aufschrauben entgegengesetzten Drehung die Führungshülse von dem domartigen Ansatz lösen.

Weitere Merkmale und Einzelheiten der Erfindung ergeben sich aus den Ansprüchen und der nachfolgenden Beschreibung eines in den Zeichnungen dargestellten Ausführungsbeispiels des Gegenstandes der Erfindung. Es zeigen:
- Fig. 1: in einem Längsschnitt eine Blutentnahmevorrichtung vor dem Zusammenfügen einer eine Doppelkanüle tragenden Führungshülse mit an ihrem Innenmantel ausgebildeten Innengewindesegmenten und einem durch eine aufgeschraubte Kappe verschlossenen, teilweise dargestellten Entnahmeröhrchen;
- Fig. 2: als Einzelheit der Fig. 1 in einer teilgeschnittenen Vorderansicht das durch die im Ausführungsbeispiel aufgeschraubte Kappe verschlossene Entnahmeröhrchen, wobei der Außenmantel des domartigen Ansatzes der Kappe mit Außengewindesegmenten ausgebildet ist;
- Fig. 3: in einer vergrößerten perspektivischen Gesamtansicht als Einzelheit der Fig. 1 die ohne Doppelkanüle dargestellte Führungshülse mit in einem unteren Abschnitt ihres Innenmantels ausgebildeten Innengewindesegmenten; und
- Fig. 4: die Führungshülse der Fig. 3 in einem Längsschnitt.

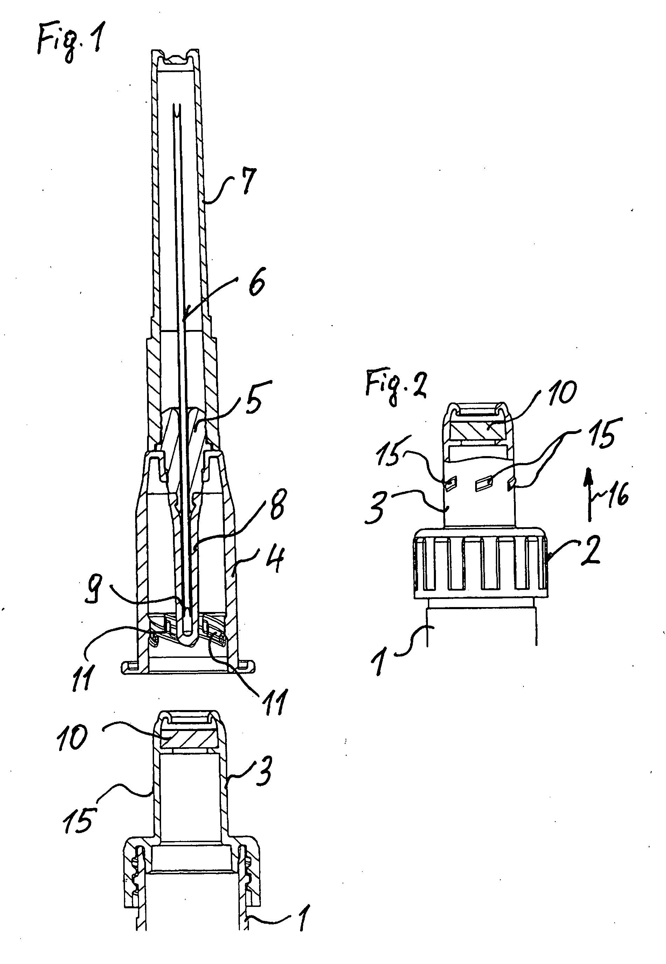

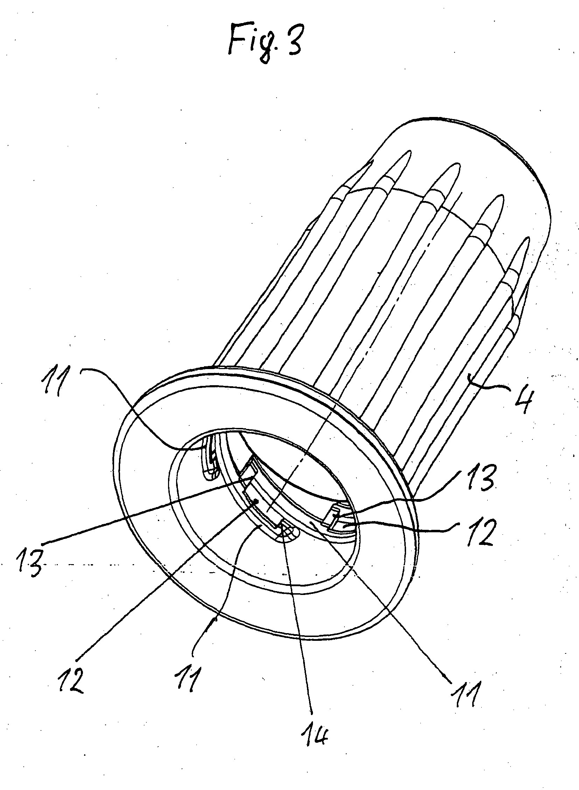

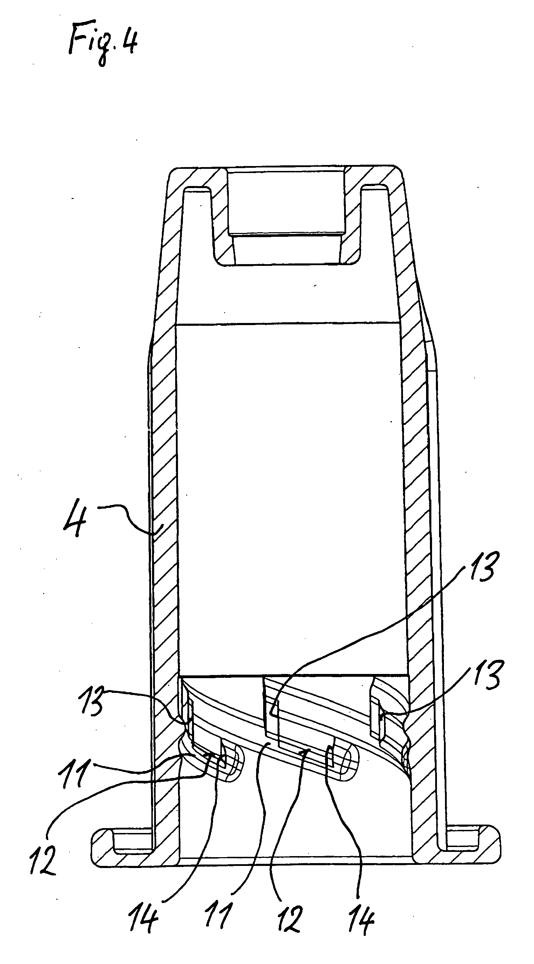

Eine im Zusammenbau nicht dargestellte Blutentnahmevorrichtung umfasst ein in den Fig. 1 und 2 nur auszugsweise angedeutetes Entnahmeröhrchen 1 mit einer dessen oberes, offenes Ende verschließenden, aufschraubbaren Kappe 2, die einen domartigen Ansatz 3 aufweist, und eine auf den Ansatz 3 aufsteckbare, aus einem verformungssteifen, harten Kunststoffmaterial bestehende in den Fig. 3 und 4 als Einzelheit dargestellte Führungshülse 4. Wie sich der Fig. 1 entnehmen lässt, trägt ein oben in die Führungshülse 4 eingesetzter Nadel- bzw. Kanülenhalter 5 eine an beiden Enden angeschärfte Doppelkanüle 6. Das oben aus dem Kanülenhalter 5 bzw. aus der Führungshülse 4 hinausragende Ende der Doppelkanüle wird von einem zum Gebrauch abziehbaren Schutzgehäuse 7 eingeschlossen, während das hintere Kanülenende über den Kanülenhalter 5 hinaus in die Führungshülse 4 hineinragt; es wird von einem schlauchartigen Ventilgummi 8 solcher Länge umschlossen, dass die Schneidkante 9 des hinteren Kanülenendes bei gestrecktem Ventilgummi 8 noch nicht dessen Boden berührt.

Zum Gebrauch der Blutentnahmevorrichtung wird die Führungshülse 4 der Fig. 1 bzw. 3 und 4 auf den domartigen Ansatz 3 der Kappe 2 aufgesteckt. Dabei tritt zunächst der Boden des Ventilgummis 8 auf einen vertieft, zurückversetzt in dem Ansatz 3 angeordneten Stopfen 10. Beim weiteren Aufstecken schiebt sich das Ventilgummi 8 zieharmonikaartig zusammen (vgl. beispielsweise Fig. 2 der eingangs genannten EP 1 455 650 B1), wird dann von der Schneidkante 9 des hinteren Kanülenendes durchstochen, die danach den Stopfen bzw. die Membrane 10 durchdringt, so dass die Schneidkante 9 der Doppelkanüle 6 zur Blutentnahme frei im Inneren des Entnahmeröhrchens 1 liegt.

Damit das Aufstecken - und gleichermaßen das Abziehen - der Führungshülse 4 auf den bzw. von dem Ansatz 3 ruckfrei, in einer für einen Patienten sehr schonenden Weise erfolgen kann und sich die Führungshülse und die Kappe 2 in einfacher Weise herstellen und montieren bzw. zusammenfügen lassen, sind die Führungshülse 4 und der Ansatz 3 mit sich komplementierenden Verriegelungs- und Haltemitteln ausgebildet. Die Führungshülse 4 weist hierzu an ihrem unteren, beim Aufstecken auf den domartigen Ansatz 3 voreilenden Ende mehrere mit gleicher Steigung am Innenmantel umfangsverteilt radial vorspringende, einander überlappende Innengewindesegmente 11 auf, die in einem Bereich angeordnet sind, der unter dem zusammengedrückten Ventilgummi 8 liegt. Benachbart zu den Innengewindesegmenten 11 sowie in diese etwas übergehend und über eine Teillänge parallel zu diesen verlaufend sind taschenartige Vertiefungen 12 ausgebildet, an deren beiden Stirnenden ein erster Anschlag 13 und ein zweiter Anschlag 14 vorgesehen ist bzw. deren Stirnenden einen solchen Anschlag bereitstellen (vgl. die Fig. 3 und 4).

Am Außenmantel des domartigen Ansatzes 3 sind, wie in Fig. 2 dargestellt, umfangsverteilt radial vorspringende, voneinander beabstandete Außengewindesegmente 15 ausgebildet. Deren Lage bzw. Anordnung auf dem Ansatz 3 ist so ausgelegt, dass die Führungshülse 4 beim Aufschieben auf den Ansatz 3 so lange von dem oberhalb der Außengewindesegmente 15 liegenden zylindrischen Außenmantel des Ansatzes 3 axial bzw. linear geführt wird, bis die hintere Schneidkante 9 der Doppelkanüle 6 das Ventilgummi 8 und danach sogleich den Stopfen bzw. die Membrane 10 durchstochen hat. Danach gleiten mit dann erst einsetztender Drehbewegung der Führungshülse 4 die Innengewinde- und Außengewindesegmente 11,15 übereinander, bis die Außengewindesegmente 15 auf die in Drehrichtung vorderen, ersten Anschläge 13 der taschenartigen Vertiefungen 12 treffen. Die Aufschraub- bzw. Drehbewegung ist damit beendet. Ein Überdrehen wird durch die ersten Anschläge 13 verhindert. Mit dem Ende der Drehbewegung kann sich das komprimierte Ventilgummi 8 etwas entspannen und schiebt die Führungshülse 4 auf den domartigen Ansatz 3 der Kappe 2 entgegen der Aufschraubbewegung, wie gemäß Pfeil 16 in Fig. 2 angedeutet, geringfügig zurück bzw. nach vorne, bis die Außengewindesegmente 15 in die taschenartigen Vertiefungen 12 eintauchen bzw. einrasten und dort in einer gesicherten Stellung zur Ruhe kommen. Ein Zurückdrehen oder versehentliches Herausrutschen aus dieser Position wird durch die hinteren, zweiten Anschläge 14 verhindert.

Wenn diese gesicherte Betriebsposition gelöst und damit die Führungshülse von der Kappe 2 abgenommen bzw. entfernt werden soll, braucht lediglich die Führungshülse wieder etwas in Gegenrichtung zum Pfeil 16, d.h. in Aufschraubbewegung linear bewegt zu werden, so dass die Außengewindesegmente 15 aus den taschenartigen Vertiefungen 12 herausrutschen können. Anschließend lässt sich die Führungshülse 4 durch eine Drehbewegung entgegengesetzt zur Aufschraubbewegung von dem domartigen Ansatz 3 lösen und abziehen.

### Bezugszeichenliste:

- 1: Entnahmeröhrchen
- 2: Kappe
- 3: domartiger Ansatz
- 4: Führungshülse
- 5: Nadel-/Kanülenhalter
- 6: Doppelkanüle
- 7: Schutzgehäuse
- 8: Ventilgummi
- 9: hintere Schneidkante/hinteres Kanülenende
- 10: Stopfen
- 11: Innengewindesegment
- 12: taschenartige Vertiefung
- 13: erster Anschlag
- 14: zweiter Anschlag
- 15: Außengewindesegment
- 16: Pfeil (zur Aufschraubbewegung entgegengerichtete Bewegung)

## Patentansprüche

1. Vorrichtung zur Entnahme von Körperflüssigkeiten, insbesondere Blut, mit einer verformungssteifen Führungshülse (4) und einem Entnahmeröhrchen (1), das an seinem vorderen Ende mit einer Kappe (2) verschlossen ist, die mit einem domartigen Ansatz (3) und einem in diesem angeordnetem durchstechbaren Stopfen (10) für die aufsteckbare Führungshülse (4) versehen ist, die auf der dem Ansatz (3) zugewandten Seite eine von einem sich beim Aufstecken der Führungshülse (4) oberhalb des Ansatzes harmonikaartig zusammendrückenden Ventilgummi (8) umschlossene Kanüle und auf der dem Ansatz (3) abgewandten Seite ein Verbindungsstück oder das vordere Ende der Kanüle, insbesondere Doppelkanüle (6), aufweist, wobei der Ansatz (3) und die Führungshülse (4) mit sich zu einer der Rückstellkraft des beim Aufstecken harmonikaartig zusammengedrückten Ventilgummis widerstehenden Verriegelung der Führungshülse (4) auf dem Ansatz (3) ergänzenden Mitteln ausgebildet sind,
**dadurch gekennzeichnet,**
**dass** am Außenmantel des Ansatzes (3) umfangsverteilt radial vorspringende, voneinander beabstandete Außengewindesegmente (15) und am Innenmantel der Führungshülse (4) umfangsverteilt radial vorspringende, einander überlappende Innengewindesegmente (11) ausgebildet sind, wobei über eine Teillänge und parallel zu den Innengewindesegmenten (11) verlaufend taschenartige, stirnendseitig von jeweils einem ersten und einem zweiten Anschlag (13 bzw. 14) begrenzte Vertiefungen (12), in die in der Endlagenposition der Führungshülse (4) die Außengewindesegmente (15) des Ansatzes (3) eingreifen, ausgebildet sind.

2. Verfahren zum Montieren einer Vorrichtung zur Entnahme von Körperflüssigkeit, insbesondere Blut, wobei die Vorrichtung aus einer verformungssteifen Führungshülse (4) und einem Entnahmeröhrchen (1) besteht, das an seinem vorderen Ende mit einer Kappe (2) verschlossen ist, die mit einem domartigen Ansatz (3) und einem in diesem angeordnetem durchstechbaren Stopfen (10) für die aufsteckbare Führungshülse (4) versehen ist, die auf der dem Ansatz (3) zugewandten Seite eine von einem sich beim Aufstecken der Führungshülse (4) oberhalb des Ansatzes harmonikaartig zusammendrückenden Ventilgummi (8) umschlossene Kanüle und auf der dem Ansatz (3) abgewandten Seite ein Verbindungsstück oder das vordere Ende der Kanüle, insbesondere Doppelkanüle (6), aufweist, und wobei der Ansatz (3) und die Führungshülse (4) mit sich zu einer der Rückstellkraft des beim Aufstecken harmonikaartig zusammengedrückten Ventilgummis widerstehenden Verriegelung der Führungshülse (4) auf dem Ansatz (3) ergänzenden Mitteln ausgebildet sind,
**dadurch gekennzeichnet,**
**dass** der domartige Ansatz (3) der Kappe (2) mit einer linearen Bewegung in die Führungshülse (4) eingeschoben wird, bis nach dem Durchstechen des Stopfens (10) des Ansatzes (3) mittels des hinteren, dann aus dem zusammengedrückten Ventilgummi (8) vorstehenden Kanülenendes (9) umfangsverteilt radial vorspringende, voneinander beabstandete Außengewindesegmente (15) am Außenmantel des Ansatzes (3) auf radial vorspringende, einander überlappende Innengewindesegmente (11) am Innenmantel der Führungshülse (4) auftreffen, dass danach mit einer Schraubbewegung der domartige Ansatz (3) und die Führungshülse (4) weiter zusammengefügt werden, wobei die Schraubbewegung von in Drehrichtung liegenden ersten Anschlägen (13) von über eine Teillänge und parallel zu den Innengewindesegmenten (11) verlaufenden taschenartigen Vertiefungen (12) begrenzt wird und mit dem Ende der Drehbewegung unter der Kraft des zusammengedrückten, komprimierten Ventilgummis (8) die Führungshülse (4) entgegen der Richtung der Aufschraubbewegung linear auf dem domartigen Ansatz (3) zurückgeschoben wird, wobei die Außengewindesegmente (15) in die Vertiefungen (12) eingerastet und in dieser Position durch den ersten Anschlägen (13) am anderen Stirnende der Vertiefungen (12) gegenüberliegenden zweiten Anschlägen (14) gegen ein Zurückdrehen oder Herausrutschen gesichert werden.

## Claims

1. A device for removing bodily fluids, more particularly blood, with a deformation-resistant guide sleeve (4) and a small sampling tube (1) which at its front end is closed with a cap (2), which is provided with a mandrel-like attachment (3) and, arranged in the latter, a pierceable stopper (10) for the attachable guide sleeve (4), which on the side facing the attachment (3) has a cannula encompassed by a valve rubber (8), which on attaching the guide sleeve (4) compresses in a concertinaed manner above the attachment, and on the side facing away from the attachment (3) has a connection piece or the front end of the cannula, more particularly double cannula (6), wherein the attachment (3) and the guide sleeve (4) are made of means that supplement the locking of the guide sleeve (4) on the attachment (3) resisting the restoring force of the valve rubber compressed in an concertinaed manner,
**characterised in**
**that** distributed around the circumference of the outer sheath of the attachment (3) are radially projecting, spaced, outer thread segments (15) and distributed around the circumference of the inner sheath of the guide sleeve (4) are radially projecting, overlapping, inner thread segments (11), wherein running over a partial length and in parallel to the inner thread segments (11), there are pocket-like recesses (12), delimited on the front side by a first and a second stop (13, 14) respectively, into which in the end position of the guide sleeve (4) the outer thread segments (15) of the attachment (3) engage.

2. A method of assembling a device for the removal of bodily fluids, more particularly blood, wherein the device comprises a deformation-resistant guide sleeve (4) and a small sampling tube (1) which at its front end is closed with a cap (2), which is provided with a mandrel-like attachment (3) and, arranged in the latter, a pierceable stopper (10) for the attachable guide sleeve (4), which on the side facing the attachment (3) has a cannula encompassed by a valve rubber (8), which on attaching the guide sleeve (4) compresses in a concertinaed manner above the attachment, and on the side facing away from the attachment (3) has a connection piece or the front end of the cannula, more particularly double cannula (6), wherein the attachment (3) and the guide sleeve (4) are made of means that supplement the locking of the guide sleeve (4) on the attachment (3) resisting the restoring force of the valve rubber compressed in an concertinaed manner
**characterised in**
**that** the mandrel-like attachment (3) of the cap (2) is pushed into the guide sleeve (4) with a linear movement until after piercing of the stopper (10) of the attachment (3) by means of the rear cannula end (9), which then projects out of the concertinaed valve rubber (8), circumferentially distributed, radially projecting, spaced, outer thread segments (15) on the outer sheath of the attachment (3) come into contact with radially projecting, overlapping inner thread segments (11) on the inner sheath of the guide sleeve (4) so that thereafter, with a screwing movement the mandrel-like attachment (3) and guide sleeve (4) are further joined together, wherein the screwing movement is limited by first stops (13), located in the direction or turning, of pocket-like recesses (12) running over a partial length and in parallel with the inner thread segments (11), and at the end of the turning movement, through the force of the concertinaed, compressed valve rubber (8), the guide sleeve (4) is pushed back against the direction of the screwing movement linearly on the mandrel-like attachment (3), wherein the outer thread segments (15) engage in the recesses (12) and in this position are secured against turning back or slipping out by the second stops (14) opposite the first stops (13) on the other end of the recesses (12).

## Revendications

1. Dispositif de prélèvement de liquides physiologiques, en particulier de sang, comportant un manchon de guidage indéformable (4) et un tube de prélèvement (1) qui est fermé à son extrémité avant par un capuchon (2) qui est pourvu d'un embout en forme de dôme (3) et d'un bouchon transperçable (10) et disposé dans celui-ci (3) pour le manchon de guidage enfichable (4) qui présente, sur sa face tournée vers l'embout (4), une canule entourée d'une soupape en caoutchouc (8) se comprimant à la manière d'un harmonica lors du fichage du manchon de guidage (4) au-dessus de l'embout et, sur sa face détournée de l'embout (3), une pièce de connexion ou l'extrémité avant de la canule (6), en particulier double canule, l'embout (3) et le manchon de guidage (4) étant constitués de moyens se complétant pour un verrouillage du manchon de guidage (4) résistant à la force de rappel de la soupape en caoutchouc se comprimant à la manière d'un harmonica lors du fichage sur l'embout (3),
**caractérisé en ce**
**que** sur l'enveloppe extérieure de l'embout (3), des segments de filetage extérieur (15) saillant radialement et répartis sur la circonférence et, sur l'enveloppe intérieure du manchon de guidage (4), des segments de filetage intérieur saillant radialement et se chevauchant (11) sont réalisés, des creux (12) en forme de poches s'étendant parallèlement aux segments de filetage intérieur (11) et limités respectivement à leur face avant par une première et une seconde butée (13 ou 14), dans lesquels creux, dans la position de fin de course du manchon de guidage (4), les segments de filetage extérieur (15) de l'embout (3) s'engrènent.

2. Procédé de montage d'un dispositif de prélèvement de liquides physiologiques, en particulier de sang, le dispositif étant composé d'un manchon de guidage indéformable (4) et d'un tube de prélèvement (1) qui est fermé à son extrémité avant par un capuchon (2) qui est pourvu d'un embout en forme de dôme (3) et d'un bouchon transperçable (10) pour le manchon de guidage enfichable (4) qui présente, sur sa face tournée vers l'embout (3), une canule entourée d'une soupape en caoutchouc (8) se comprimant à la manière d'un harmonica lors du fichage du manchon de guidage (4) au-dessus de l'embout et, sur sa face détournée de l'embout (3), une pièce de connexion ou l'extrémité avant de la canule, en particulier double canule (6), l'embout (3) et le manchon de guidage (4) étant constitués de moyens se complétant pour un verrouillage du manchon de guidage (4) résistant des moyens se complétant pour un verrouillage résistant à la force de rappel de la soupape en caoutchouc se comprimant à la manière d'un harmonica lors du fichage du manchon de guidage (4) sur l'embout (3),
**caractérisé en ce**
**que** l'embout en forme de dôme (3) du capuchon (2) est glissé par un mouvement linéaire dans le manchon de guidage (4) jusqu'à ce que, une fois que le bouchon (10) de l'embout (3) est transpercé par l'extrémité de la canule (9) saillant alors de la soupape en caoutchouc comprimée (8), des segments de filetage extérieur espacés mutuellement (15) se posent sur l'enveloppe extérieure de l'embout (3) sur des segments du filetage intérieur saillant radialement et se chevauchant (11) sur l'enveloppe intérieure du manchon de guidage (4), que l'embout en forme de dôme (3) et le manchon de guidage (4) sont ensuite également assemblés, le mouvement de vissage de premières butées (13) situées dans le sens de rotation est limité par des creux en forme de poches (12) s'étendant sur une longueur partielle et parallèlement aux segments de filetage intérieur (11) et que, à la fin du mouvement de rotation, sous la force de la soupape en caoutchouc comprimée et compactée (8), le manchon de guidage (4) est reculé dans le sens inverse au sens de vissage linéairement sur l'embout en forme de dôme (3), les segments de filetage extérieur (15) s'engrenant dans les creux (12) et étant bloqués dans cette position par les secondes butées (14) opposées aux premières butées (13) à l'autre extrémité avant des creux (12) pour éviter toute rotation vers l'arrière ou tout glissement vers l'extérieur.
